# FASCICULE DE BREVET EUROPEEN

(11) **EP 4 110 202 B1**
(45) Date de publication et mention de la délivrance du brevet: **05.08.2026**
(21) Numéro de dépôt: 21705951.8
(22) Date de dépôt: 18.02.2021
(51) Int. Cl.: A61B 17/16, A61B 46/10, A61B 17/00, A61B 10/02

(54) **COUPLEUR ET DISPOSITIF CHIRURGICAL À COLLERETTE DE RECOUVREMENT D'UNE ENVELOPPE SOUPLE STÉRILE**
KOPPLER UND CHIRURGISCHE VORRICHTUNG MIT EINEM FLANSCH ZUM ABDECKEN EINER STERILEN FLEXIBLEN HÜLLE
COUPLER AND SURGICAL DEVICE WITH A FLANGE FOR COVERING A STERILE FLEXIBLE WRAPPER

(30) Priorité: 24.02.2020 FR 2001807
(43) Date de publication de la demande: 04.01.2023
(73) Titulaire: Varian Medical Systems, Inc., Palo Alto, CA 94304 (US)
(72) Inventeur: Fumex, Laurent, Madison, CT 06443 (US); Masseglia, Thierry, 83130 La Garde (FR)
(74) Mandataire: Mathisen & Macara LLP
(86) Numéro de dépôt international: PCT/EP2021/053975
(87) Numéro de publication internationale: WO 2021/170475

(56) Documents cités:
- WO-A1-2017/046531
- US-A1- 2008 045 860
- US-A1- 2009 194 446
- US-A1- 2017 027 658
- US-A1- 2017 325 791
- US-B1- 6 309 358

## Description

L'invention se rapporte au domaine général de l'instrumentation chirurgicale percutanée. Elle se rapporte plus particulièrement aux instruments chirurgicaux percutanés destinés à traverser l'épiderme et les parties molles du corps afin d'effectuer, par exemple, des prélèvements de moelle et de tissus osseux à des fins de biopsie.

Dans le domaine de la chirurgie percutanée et plus particulièrement celui de la biopsie de moelle et de la biopsie osseuse percutanée, il est connu d'utiliser différents instruments permettant à la fois de pénétrer la peau puis les tissus mous puis la corticale de l'os concerné et de réaliser le prélèvement de substances organiques (moelle osseuse, échantillons osseux) contenues dans l'os ou dans sa cavité.

Ces instruments sont généralement connus sous l'appellation d'aiguilles ou de trocarts à biopsie de moelle ou à biopsie osseuse et comprennent généralement un trocart ou une aiguille d'accès, pouvant être combinés à une canule biopsique. De nature plus ou moins perforante, les trocarts ou aiguilles d'accès permettent au praticien de traverser les tissus mous puis de forer l'os considéré de façon à atteindre une zone dite de prélèvement.

Ces trocarts comportent généralement un tube externe creux, dont l'extrémité est plus ou moins coupante, et à l'intérieur duquel coulisse une tige appelée mandrin ou stylet dont l'extrémité est affûtée de façon à perforer la corticale de l'os. Le prélèvement de tissu osseux ou de moelle peut être réalisée par cette tige elle-même ou par le tube externe creux. Dans ce cas, la tige est provisoirement retirée du tube et celui-ci est poussé ou vissé en avant afin de capturer le prélèvement. Dans une autre configuration, une canule biopsique de forme variée est introduite dans le tube en lieu et place de la tige et afin d'effectuer le prélèvement de moelle ou d'os.

De tels instruments sont généralement mis en œuvre de manière manuelle au moyen d'une poignée solidarisée, de manière permanente ou non, à l'extrémité proximale (extrémité n'entrant pas en contact avec l'os) de la tige perforante, que le praticien actionne en rotation de façon à forer l'os. En pratique, le praticien amène l'extrémité du tube externe du trocart au contact de l'os puis actionne la poignée en rotation pour faire pénétrer l'extrémité de la tige dans l'os.

Ainsi, la demande Internationale publiée sous la référence WO2006/061514 décrit un trocart destiné à la biopsie osseuse comprenant un tube externe, dont l'extrémité distale est divisée en deux segments à bord coupant hélicoïdal, dans lequel coulisse une tige affûtée. Ce type d'instrument est destiné à être mis en œuvre manuellement au moyen d'une poignée.

La mise en œuvre manuelle d'un tel trocart, au moyen d'une poignée directement actionnée par le praticien, correspond à un mode opératoire classique qui permet à ce dernier de maitriser de manière fine le geste de perforation de l'os. Cependant, dans certaines circonstances, en particulier lorsque la dureté de l'os requiert une plus grande vigueur pour faire pénétrer l'aiguille à travers la paroi de l'os, il est alors parfois plus confortable, tant pour le patient que pour le praticien, de disposer d'un outil d'entrainement, de type motorisé, pour faire pénétrer la tige à l'intérieur de l'os sans appliquer une force excessive.

Par suite il y a un grand avantage pour le praticien à pouvoir disposer d'un trocart dont la tige perforante peut être actionnée indifféremment au moyen d'une simple poignée ou par l'intermédiaire d'un outil d'entrainement.

Une telle possibilité permet avantageusement au praticien, de débuter une procédure opératoire manuellement puis, si la dureté de l'os le nécessite, de terminer la perforation au moyen d'un entrainement automatique en rotation et ce, sans qu'il soit nécessaire de changer la tige perforante et donc sans risque de perte du point d'entrée dans l'os.

Pour cela, un dispositif d'interface capable par une de ses extrémités de venir se substituer à la poignée utilisée par le praticien peut être utilisé pour une mise en œuvre manuelle du trocart ; en se montant par exemple à l'extrémité de ce dernier de manière identique à la poignée, et portant à son autre extrémité un élément d'interface sur lequel l'embout d'un outil d'entrainement puisse venir se fixer de manière amovible.

Le dispositif de la présente invention concerne un élément de couplage qui permet d'entrainer le trocart en rotation au moyen d'un outil.

L'objectif d'un tel dispositif est, entre autres fonctionnalités, de pouvoir assurer un positionnement axial relativement précis de l'outil d'entrainement vis-à-vis du trocart, faute de quoi la précision de pénétration du trocart pourrait se trouver affectée.

De plus, le dispositif d'interface est destiné à assurer le raccordement de l'extrémité d'un trocart, objet stérile et généralement à usage unique, à un outillage de type perceuse, objet naturellement réutilisable et généralement non stérile. Dès lors, il est préférable que le dispositif d'interface puisse être associé de manière simple à des moyens permettant le montage de l'outil d'entrainement à l'extrémité du trocart et l'entrainement en rotation dudit trocart en évitant tout contact entre le trocart et l'outil d'entrainement, de nature à provoquer la contamination de l'un par l'autre.

Le brevet US 6,716,215 B1 décrit un moyen offrant une solution pour raccorder une mèche stérile à une perceuse non stérile au travers d'un sachet stérile. Le sachet stérile, sur lequel est fixée une bague, est destiné à recevoir une perceuse qu'il permet d'emballer pour pouvoir l'utiliser dans un milieu stérile.

Dans la zone de raccordement de la perceuse à la mèche, l'étanchéité est réalisée au moyen d'un joint torique, logé dans la bague, qui fait étanchéité sur le corps de la mèche. L'étanchéité est réalisée par compression du joint.

Cette solution présente cependant l'inconvénient que le sachet peut se trouver entrainé en rotation du fait des frottements du joint d'étanchéité sur la mèche et qu'il peut donc, en tournant, bloquer la perceuse en rotation.

Le brevet US 7,850,620 B2 décrit un système permettant de raccorder un dispositif motorisé, non stérile et logé dans un sachet stérile, à un instrument de biopsie stérile au moyen d'un coupleur solidaire du sachet. Le coupleur assemblé comporte une bague bloquée en translation mais libre en rotation sur laquelle est soudé le sachet. Cette solution ne permet pas de réaliser une étanchéité complète car la bague peut laisser passer des impuretés de par son jeu de fonctionnement.

Par ailleurs, un dispositif tel que décrit par le brevet US 7,850,620 B2 n'exclut pas le risque que compte-tenu de la structure du coupleur assemblé, le sachet ne soit entrainé en rotation sous l'effet de frottements et ne bloque ainsi la perceuse en rotation en se resserrant autour d'un élément en rotation.

Le dispositif décrit dans le brevet US 7,850,620 B2 ne permet pas d'assurer une bonne concentricité de l'ensemble lors de l'utilisation par le praticien de par le cumul des jeux fonctionnels entre les différents assemblements. Le jeu fonctionnel de l'instrument monté dans le coupleur assemblé ajouté aux jeux fonctionnels des différents composants du coupleur assemblé ajoutés au jeu fonctionnel du coupleur assemblé monté sur l'arbre moteur de l'outil d'entrainement font qu'au final l'extrémité de l'instrument est instable par rapport à l'outil d'entrainement, ce qui est gênant pour le praticien et pour la précision de l'acte chirurgical.

Enfin, le nombre de pièces composant ce dispositif ainsi que leur agencement relatif entrainent des coûts de fabrication élevés.

La demande de brevet EP 1 771 034 A1 décrit un système permettant de raccorder un dispositif motorisé type perceuse non stérile, logé dans un sachet stérile, à un instrument de biopsie stérile au moyen d'un coupleur. Le sachet comporte une bague dont la forme permet d'épouser la forme du nez de la perceuse. Un ergot sur le nez de la perceuse en forme de clavette et un bossage de la bague permettent de solidariser la bague à la perceuse et de bloquer toute rotation du sac autour de la perceuse. Ainsi lorsque la perceuse munie de son coupleur est placée dans le sachet, la bague et le sachet sont bloquées en rotation par l'ergot en forme de clavette et ce en dépit du frottement du joint d'étanchéité du sachet sur le corps du coupleur. Cette solution permet de réaliser tout à la fois une étanchéité du sachet et de réaliser le blocage en rotation de celui-ci.

Cependant, le dispositif motorisé étant réutilisable exige un nettoyage méticuleux de celui-ci difficile à réaliser en présence d'ergots en forme de clavette ou tout autre protubérance présentant des arêtes plus ou moins vives. De plus l'embout de couplage de la perceuse comporte des usinages laissant des zones difficiles à nettoyer et le corps de la perceuse ne recouvrant pas l'embout de couplage, rien n'exclut que des impuretés ne puisse être éjectées sous l'effet de la force centrifuge générée par le mouvement rotatif de la perceuse combiné au vieillissement du joint d'étanchéité du sachet sous l'effet du frottement et de la chaleur ainsi dégagée. L'orientation du repli du joint d'étanchéité vers l'instrument chirurgical stérile peut faciliter l'éjection d'impuretés vers le patient lors de l'intervention sous l'effet combiné de la force centrifuge, du vieillissement du joint et de l'air prisonnier du sachet qui sortirait par le joint. Ce mode de réalisation demande une bonne concentricité de l'ouverture réalisée dans le sachet par rapport à la bague pour éviter une ovalisation du repli autour du corps du coupleur. Cette précision est difficile à obtenir car le sachet est fabriqué avec des matériaux souple et donc très déformable.

Il existe donc un besoin pour un dispositif de couplage d'un outil d'entraînement à un instrument chirurgical percutané n'ayant pas les inconvénients précités.

Les documents US 2008/045860 A1, qui représente l'art antérieur le plus proche, et US 2009/194446 A1 concerne un embout de connexion d'un outil d'entrainement.

La présente invention a pour but de fournir un dispositif de couplage de conception simple et peu couteuse permettant également de garantir une bonne étanchéité entre les éléments stériles et non stériles couplés ensemble.

La présente invention a également pour but de garantir une bonne concentricité et un bon alignement entre l'instrument chirurgical percutané et l'outil d'entraînement de manière à obtenir un positionnement précis de l'instrument.

La présente invention a en outre pour but de limiter les jeux de fonctionnement induits par le dispositif de couplage pour garantir un positionnement précis de l'instrument chirurgical et ainsi faciliter son utilisation.

Pour cela, l'invention concerne un coupleur pour le couplage stérile d'un instrument chirurgical percutané à un outil d'entrainement en rotation, s'étendant le long d'un axe de couplage, ledit coupleur comprenant :
- une portion distale configurée pour être connectée de manière amovible à un instrument chirurgical percutané,
- une portion proximale configurée pour être connectée de manière amovible à un outil d'entrainement,
- une portion centrale disposée entre les portions distale et proximale, la portion centrale comprenant :
   ∘une collerette d'appui s'étendant transversalement à l'axe de couplage et configurée pour déformer axialement une extrémité distale d'une enveloppe souple entourant l'outil d'entraînement lorsque la portion proximale est connectée à cet outil d'entraînement,
   ∘ une collerette de maintien s'étendant le long de l'axe de couplage au moins partiellement en recouvrement de la portion proximale de manière à pouvoir maintenir l'extrémité distale de l'enveloppe souple stérile entre la collerette de maintien et le boîtier d'un outil d'entraînement lorsque la portion proximale est connectée à l'outil d'entraînement
dans lequel le coupleur forme un accessoire unitaire dans lequel les portions distale, proximale et centrale sont solidaires les unes des autres.

L'agencement des collerettes d'appui et de maintien permet de former un important obstacle à la propagation des impuretés entre la portion distale du coupleur à protéger des impuretés et la portion proximale au niveau de laquelle des impuretés peuvent être générées, notamment depuis un embout de connexion de l'outil d'entrainement.

Cet obstacle à la propagation des impuretés est à la fois transversal par rapport à l'axe de rotation du coupleur, au moyen de la collerette d'appui, et longitudinal à cet axe de rotation, au moyen de la collerette de maintien.

De plus, la déformation de l'extrémité de l'enveloppe souple par la collerette d'appui permet d'utiliser l'extrémité de l'enveloppe souple comme un obstacle supplémentaire à la propagation des impuretés au niveau de la portion proximale du coupleur. Ceci accroit encore davantage la difficulté des impuretés à s'extraire de l'extrémité de l'outil d'entraînement vers la portion distale du coupleur destinée à être disposée au niveau d'un environnement stérile.

Selon un mode de réalisation du coupleur, les portions distale (27), proximale (28) et centrale (29) sont fixes les unes par rapport aux autres
Selon un mode de réalisation du coupleur, la collerette de maintien s'étend autour de l'axe de couplage de manière à former une cloche de protection configurée pour recouvrir une extrémité de connexion d'un outil d'entraînement.

Selon un mode de réalisation du coupleur, la portion centrale comprend un corps de liaison disposé entre les portions distale et proximale, la collerette d'appui s'étendant à partir du corps de liaison et formant avec la collerette de maintien une forme générale de U

Selon un mode de réalisation du coupleur, la collerette de maintien s'étend à partir d'une extrémité de la collerette d'appui pour former un espace annulaire autour de la portion centrale. Les collerettes d'appui et de maintien forment ainsi une paroi recourbée vers la portion proximale et donc vers l'outil d'entrainement lorsque celui-ci est connecté au coupleur. Cette paroi recourbée permet ainsi d'éviter que les potentielles impuretés provenant de l'outil d'entrainement ne soient projetées vers la portion distale où se situe l'environnement stérile.

Les collerettes d'appui et de maintien forment ainsi une jupe de protection recouvrant au moins partiellement la portion proximale et donc l'extrémité de l'outil d'entrainement.

Selon un mode de réalisation du coupleur, la portion centrale comprend en outre une collerette de protection s'étendant le long de l'axe de couplage entre la collerette de maintien et la portion proximale. Une barrière supplémentaire est ainsi formée en travers du passage des potentielles impuretés provenant de l'outil d'entraînement. Ceci permet d'augmenter les chances de stopper la propagation de ces impuretés de manière à limiter encore davantage les risques de contamination de l'environnement stérile. La collerette de protection peut également s'étendre de manière à recouvrir au moins partiellement la portion proximale. Ainsi, l'obstacle formé par la collerette de protection est encore plus significatif et forme un virage supplémentaire dans le chemin pris par les potentielles impuretés.

Selon un mode de réalisation du coupleur, la portion proximale est une connexion mâle configurée pour se connecter à un embout de connexion femelle d'un outil d'entraînement.

L'invention concerne également un dispositif chirurgical stérile pour l'entrainement en rotation d'un instrument chirurgical percutané, comprenant :
- un coupleur selon l'une quelconque des revendications précédentes,
- une enveloppe souple formant :
   ∘ une cavité de réception d'un outil d'entraînement,
   ∘une première ouverture configurée pour permettre l'insertion de l'outil d'entraînement à l'intérieur de la cavité de réception,
   ∘ une portion d'extrémité configurée pour recevoir une extrémité de connexion de l'outil d'entraînement, la portion d'extrémité formant une deuxième ouverture débouchant à l'intérieur de la cavité de réception et configurée pour permettre l'insertion partielle du coupleur à l'intérieur de la cavité de réception au travers de la deuxième ouverture,
   ∘ une portion annulaire d'étanchéité configurée pour être reçue au contact de la surface annulaire du boîtier de l'outil d'entraînement lorsque l'extrémité de connexion de l'outil d'entraînement est insérée dans la portion d'extrémité de l'enveloppe,
la collerette de maintien du coupleur étant configurée pour recouvrir au moins partiellement la portion annulaire de l'enveloppe lorsque l'extrémité de connexion de l'outil d'entraînement est insérée dans la portion d'extrémité de l'enveloppe avec la portion proximale du coupleur connectée à l'embout de connexion.

Selon un mode de réalisation du dispositif chirurgical, celui-ci comprend en outre :
- un outil d'entraînement comprenant :
   ∘ un boîtier et un organe d'entrainement en rotation disposé à l'intérieur du boîtier, le boîtier définissant une surface annulaire de réception d'une enveloppe souple au niveau d'une extrémité de connexion de l'outil d'entraînement,
   ∘ un embout de connexion disposé au niveau de l'extrémité de connexion et configuré pour être connecté de manière amovible à la portion proximale du coupleur, l'embout de connexion étant solidaire de l'organe d'entrainement de manière à transmettre au coupleur un mouvement de rotation de l'organe d'entrainement lorsque la portion proximale est connectée à l'embout de connexion.

Selon un mode de réalisation du dispositif chirurgical, l'enveloppe est dépourvue de pièce rigide de connexion à l'outil d'entraînement ou au coupleur, à l'état déconnecté de l'outil d'entraînement, du coupleur et de l'enveloppe.

Le coût de fabrication de l'enveloppe est ainsi grandement réduit par rapport à une enveloppe au niveau de laquelle une bague est fixée, tel que décrit par exemple dans le brevet US 7,850,620 B2. De plus, la solidarisation de l'enveloppe avec l'outil d'entraînement est facilitée car l'outil d'entraînement se positionne dans la portion d'extrémité de l'enveloppe par gravité sans avoir besoin de faire correspondre des formes ou mécanismes de fixation.

Selon un mode de réalisation du dispositif chirurgical, la portion annulaire de l'enveloppe et la surface annulaire du boîtier de l'outil d'entraînement sont en complémentarité de forme. Ainsi, la portion annulaire de l'enveloppe s'ajuste sur la surface annulaire du boîtier de l'outil d'entraînement pour limiter la propagation des impuretés depuis l'intérieur vers l'extérieur de l'enveloppe.

Selon un mode de réalisation du dispositif chirurgical, la portion annulaire de l'enveloppe présente une section transversale au moins partiellement inférieure à la section transversale de la surface annulaire du boîtier de l'outil d'entraînement de manière à déformer l'enveloppe lorsque l'outil d'entrainement est inséré dans la portion d'extrémité avec la portion annulaire et la surface annulaire en contact l'une avec l'autre.

Selon un mode de réalisation du dispositif chirurgical, la portion annulaire et la surface annulaire ont une section transversale conique descendant respectivement vers la deuxième ouverture et l'embout de connexion. Cette conformation conique permet à la surface annulaire de l'outil d'entraînement de s'ajuster au plus près de la portion annulaire de l'enveloppe.

Selon un mode de réalisation du dispositif chirurgical, la portion annulaire de l'enveloppe est contiguë à la portion d'extrémité.

Selon un mode de réalisation du dispositif chirurgical, l'enveloppe est formée par au moins deux feuillets fixées entre eux au niveau de leurs bords d'extrémité pour former la cavité de réception, un retour sensiblement transversal aux feuillets étant formé le long des bords d'extrémité.

Selon un mode de réalisation du dispositif chirurgical, lesdits au moins deux feuillets sont fixés ensemble par soudage.

Selon un mode de réalisation du dispositif chirurgical, la collerette de maintien s'étend de manière à recouvrir au moins un tiers de la longueur de la portion annulaire le long de l'axe de couplage lorsque l'outil d'entrainement est connecté au coupleur. Ceci permet un bon compromis entre la surface recouverte par la collerette de maintien et la facilité de montage du coupleur. En effet, plus la collerette de maintien est longue, plus la visibilité facilitant l'orientation et l'insertion de la partie proximale du coupleur dans l'outil d'entrainement en est réduite.

Selon un mode de réalisation du dispositif chirurgical, la collerette de maintien comprend une extrémité distale disposée au niveau de la portion centrale du coupleur et une extrémité proximale libre, la collerette de maintien et la surface annulaire de l'outil d'entraînement étant dimensionnées de manière à former un jeu inférieur ou égal à 3mm, de préférence inférieur ou égal à 2mm, entre l'extrémité proximale de la collerette de maintien et la surface annulaire de l'outil d'entraînement lorsque l'outil d'entrainement est connecté au coupleur. L'espace séparant l'extrémité de connexion de l'outil d'entraînement da la collerette de maintien est ainsi très faible. Ceci empêche encore davantage la propagation des impuretés car cet espace est également occupé d'une part par l'extrémité proximale de l'enveloppe déformée par la collerette d'appui et d'autre part par l'appui des bords d'extrémité de l'enveloppe contre la face interne de la collerette de maintien.

### Brève description des dessins

Les dessins annexés illustrent l'invention :
[Fig. 1] représente une vue en coupe d'un coupleur connecté à une extrémité de connexion d'un outil d'entraînement, une enveloppe souple étant positionnée autour de l'outil d'entrainement entre une collerette de maintien du coupleur et l'extrémité de connexion de l'outil d'entrainement.
[Fig. 2] représente une vue en coupe du coupleur connecté à l'outil d'entraînement tel que visible en figure 1 dans une position angulaire différente du plan de coupe, laissant ainsi apparaître un retour transversal de l'enveloppe.
[Fig. 3] représente une vue de côté du coupleur selon la figure 1 connecté à l'outil d'entraînement, la collerette de maintien et la collerette d'appui du coupleur étant représentées en coupe.
[Fig. 4] représente une vue en perspective de l'outil d'entraînement.
[Fig. 5] représente une vue détaillée d'une extrémité de connexion de l'outil d'entraînement selon la figure 4.
[Fig. 6] représente une vue en perspective de l'outil d'entraînement selon la figure 4 sous un angle de vue différent.
[Fig. 7] représente une vue détaillée de l'extrémité de connexion de l'outil d'entraînement selon la figure 6.
[Fig. 8] représente une vue de côté d'une enveloppe souple stérile selon les figures 1 à 3 dans laquelle un outil d'entraînement est à insérer.
[Fig. 9] représente une vue en perspective d'un coupleur selon les figures 1 à 3.
[Fig. 10] représente une vue en perspective du coupleur selon les figures 1 à 3 sous un angle de vue différent.
[Fig. 11] représente une vue de côté en perspective du coupleur selon les figures 9 et 10, les collerettes d'appui et de maintien ainsi qu'une collerette de protection du coupleur étant représentées en coupe.

### Description de mode(s) de réalisation

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation. De plus, le terme « comprenant » n'exclut pas d'autres éléments ou étapes.

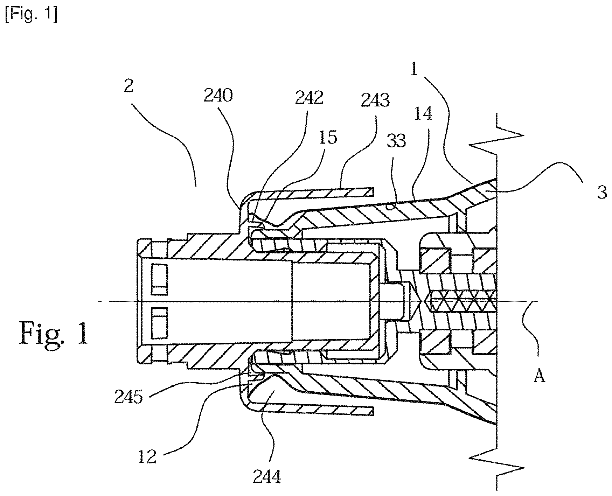

En référence aux figures 1 à 3, il est proposé un dispositif chirurgical 10 pour l'entrainement en rotation d'un instrument chirurgical percutané (non visible). Ce dispositif chirurgical 10 comprend notamment un outil d'entraînement 3 et un coupleur 2 pour le couplage de l'outil d'entraînement 3 et à un instrument chirurgical percutané. Le coupleur 2 permet de transmettre le mouvement de rotation généré par l'outil d'entrainement à l'instrument chirurgical percutané.

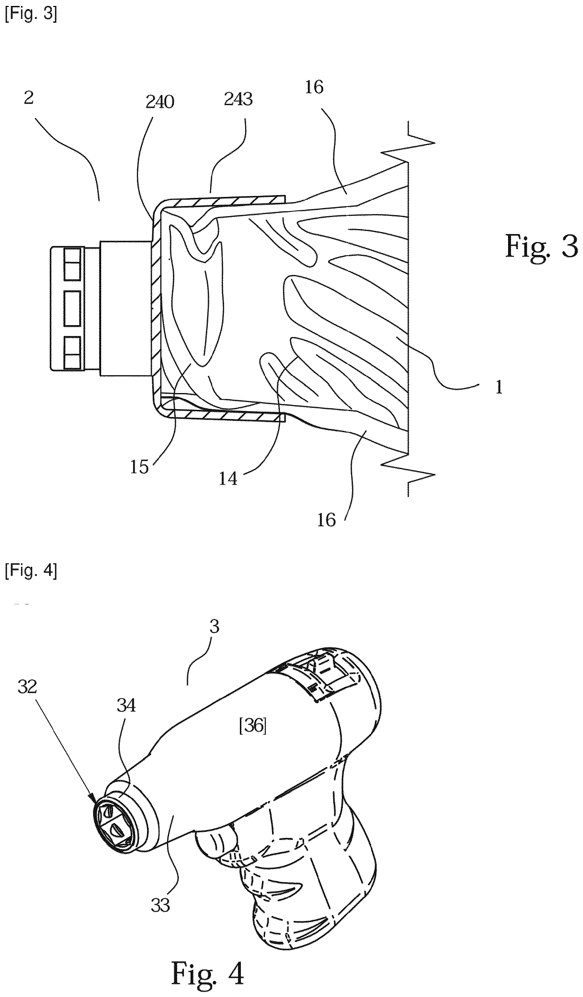

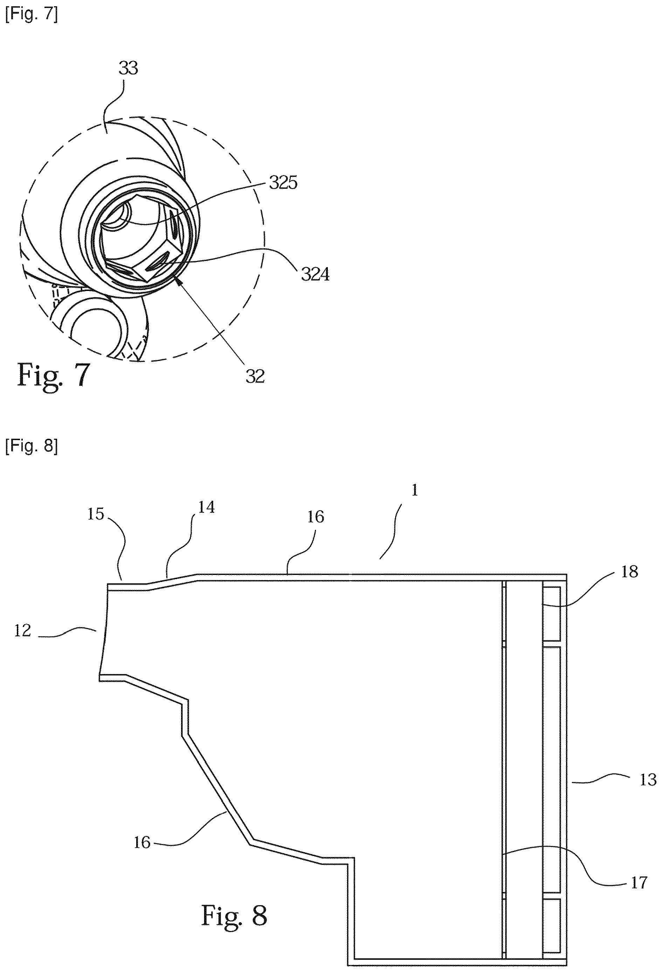

En référence aux figures 4 à 7, l'outil d'entraînement 3 comprend un boîtier 36 et un organe d'entrainement en rotation (non visible) disposé à l'intérieur de ce boîtier 36. Le boîtier 36 comprend une extrémité de connexion 31 au niveau de laquelle le coupleur 2 peut être connecté à l'outil d'entraînement 3.

L'outil d'entraînement 3 comprend également un embout de connexion 32 disposé au niveau de l'extrémité de connexion 31. L'embout de connexion 32 est configuré pour être connecté de manière amovible au coupleur 2. L'embout de connexion 32 est en outre solidaire de l'organe d'entrainement de manière à pouvoir transmettre au coupleur 2 un mouvement de rotation de l'organe d'entrainement.

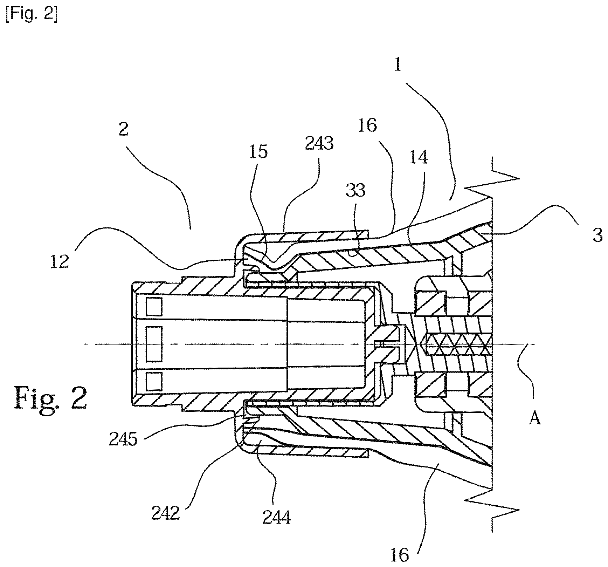

De manière préférée, l'embout de connexion 32 ne s'étend pas hors du boîtier 36 de manière à limiter les espaces dans lesquels des impuretés pourraient se loger. En d'autres termes, l'embout de connexion 32 ne sort de préférence pas de l'extrémité de connexion 31 du boîtier 36. De manière préférée, l'embout de connexion 32 est en retrait de l'extrémité distale du boîtier 36. Ceci se traduit par le fait que l'embout de connexion 32 est totalement recouvert par le boîtier 36 lorsqu'il est observé en vue de côté tel que visible sur les figures 1 et 2. Ceci permet de recouvrir toutes les parties tournantes de l'outil d'entrainement 3 et d'éviter toute projection de particules situées sur l'embout de connexion 32 lors de la rotation de celui-ci.

L'organe d'entraînement 3 comprend notamment un moteur accouplé à un organe de transmission lui-même accouplé à l'embout de connexion 32. L'organe de transmission est de préférence un organe de réduction permettant de réduire la vitesse de rotation transmise par le moteur tout en augmentant le couple transmis à l'embout de connexion 32. De manière alternative, l'outil d'entraînement 3 peut être dépourvu d'organe de réduction de sorte que l'organe de transmission permet uniquement de transmettre le mouvement de rotation du moteur à l'embout de connexion 32 sans modification de la vitesse de rotation ou du couple. Le moteur est par exemple un moteur électrique. L'organe d'entraînement peut donc intégrer à l'intérieur du boîtier 36 une batterie et/ou un port de connexion à une alimentation électrique. La batterie peut être rechargeable.

Selon l'exemple de réalisation représenté en figures 4-7, l'embout de connexion 32 se présente comme une cavité 321 comportant une paroi interne qui présente une zone arrière 323 de forme hexagonale et une extrémité avant 322 de forme tronconique. Une gorge chanfreinée 324 est réalisée dans la paroi interne de la cavité 321 au niveau de la zone arrière hexagonale 323.

Dans cet exemple, l'embout de connexion 32 comporte également un perçage cylindrique borgne 325 formant le fond de l'embout, dont l'axe de symétrie est confondu avec l'axe de symétrie général de l'embout de couplage 32.

Le dispositif chirurgical 10 comprend en outre une enveloppe souple 1 stérile destinée à recouvrir au moins partiellement l'outil d'entraînement 3 pour l'isoler de l'environnement stérile. L'enveloppe souple 1 forme une cavité de réception de l'outil d'entraînement 3. On entend par « souple » le fait que l'enveloppe 1 est apte à se déformée élastiquement sous l'action d'un utilisateur pour former un volume intérieur permettant l'insertion de l'outil d'entraînement 3 à l'intérieur de l'enveloppe 1. La souplesse de l'enveloppe 1 est notamment définie par l'épaisseur des parois qui la forment ainsi que par son matériau.

L'enveloppe 1 est de préférence formée par au moins deux feuillets fixées entre eux au niveau de leurs bords d'extrémité pour former la cavité de réception. Ces feuillets sont par exemple fixés ensemble par soudage. Un retour ou bordure 16 est formé le long des bords d'extrémité, de largeur inférieure à égale à 5mm. Ce retour 16 correspond à une bande libre formée entre le bord d'extrémité des feuillets et la zone où les feuillets ont été fixées ensemble, par exemple par soudage. Ce retour 16 est notamment visible sur les figures 2, 3 et 8. Ce retour 16 s'étend sensiblement transversalement aux parois de l'enveloppe 1 formant la cavité de réception lorsqu'un objet est inséré à l'intérieur de cette cavité de réception, notamment l'outil d'entraînement 3. L'enveloppe 1 est réalisée de préférence en matière plastique compatible avec le milieu chirurgical.

Les feuillets formant l'enveloppe 1 sont par exemple d'une épaisseur inférieure à 1mm, de préférence inférieure à 0,5mm, de manière encore préférée égale à 0,15mm.

L'enveloppe souple 1 est dépourvue de pièce rigide de connexion à l'outil d'entraînement 3 ou au coupleur 2. Dès lors, lorsque l'enveloppe 1 n'est pas montée sur l'outil d'entraînement 3 ou n'est pas à proximité du coupleur 2, par exemple lorsque le dispositif chirurgical 10 est disposé sur un plateau de conditionnement, aucune pièce rigide de connexion n'est fixée à l'enveloppe 1. En d'autres termes, à l'état déconnecté ou démonté du dispositif chirurgical 10, l'enveloppe 1 ne comprend aucune pièce de connexion rigide. Cet état déconnecté ou démonté du dispositif chirurgical 10 correspond à un état dans lequel l'enveloppe 1, le coupleur 2 et l'outil d'entraînement 3 sont dissociés les uns des autres, i.e. sans être fixés les uns aux autres.

Le coupleur 2 est un accessoire unitaire. En d'autres termes, il forme un accessoire monobloc dont les sous-ensembles sont solidaires et n'ont pas vocation à être dissociés dans l'utilisation du coupleur 2. Le coupleur 2 peut être réalisé en une seule et même pièce ou en au moins deux pièces solidarisées ensemble. Ainsi, il est possible que l'une des portions du coupleur 2 soit clipsée ou assemblée aux autres portions du coupleur 2 avant son utilisation. Dans tous les modes de réalisation, les portions du coupleur 2 n'ont pas vocations à être mobiles les unes des autres. Ainsi, le coupleur 2 est un accessoire unitaire et solidaire. Si des mobilités sont possibles entre pièces, elles sont dues uniquement au jeu de montage. Il n'est pas souhaitable qu'il y ait une mobilité entre ces portions et que des particules puissent circuler entre ces portions.

Une première ouverture 13 est formée par l'enveloppe souple 1. Cette première ouverture 13 est configurée pour permettre l'insertion de l'outil d'entraînement 3 à l'intérieur de la cavité de réception. La première ouverture 13 est dimensionnée de façon à permettre une insertion aisée de l'outil d'entrainement 3 à l'intérieur de l'enveloppe 1. La première ouverture 13 est pourvue de deux replis 17 pour faciliter la manipulation et de moyens de fermeture 18 (adhésifs par exemple) permettant de refermer la première ouverture 13 de manière étanche.

L'enveloppe souple 1 forme également une portion d'extrémité 15 configurée pour recevoir l'extrémité de connexion 31 de l'outil d'entraînement 3. En d'autres termes, l'enveloppe souple 1 comprend un rétrécissement dans lequel le nez de l'outil d'entraînement 3 peut être inséré. Un outil d'entraînement, par exemple du type perceuse, est usuellement formé d'une première portion formant une poignée et une deuxième portion à laquelle une pièce est accouplée pour être entraînée en rotation. De manière préférée, le nez de l'outil d'entraînement 3 correspond à cette deuxième portion de sorte que l'extrémité de connexion 31 est disposée au niveau du nez de l'outil d'entraînement 3.

Une deuxième ouverture 12 est formée au niveau de la portion d'extrémité et débouche à l'intérieur de la cavité de réception de l'enveloppe souple 1. Cette deuxième ouverture 12 est en particulier formée au niveau d'une extrémité distale de l'enveloppe 1. Cette deuxième ouverture 12 est configurée pour permettre l'insertion partielle du coupleur 2 à l'intérieur de la cavité de réception au travers de la deuxième ouverture 12. De manière préférée, l'insertion partielle du coupleur 2 correspond à l'insertion d'une portion proximale de connexion du coupleur à l'outil d'entraînement. Ainsi, le coupleur 2 est connecté à l'outil d'entraînement au travers de la deuxième ouverture 12 de l'enveloppe souple 1.

Cette deuxième ouverture 12 est formée de préférence à l'opposé de la première ouverture 13 par rapport à la cavité de réception de manière à faciliter le positionnement de l'outil d'entraînement 3 dans l'extrémité de connexion 15. En effet, l'outil d'entraînement 3 est de préférence inséré à l'intérieur de la cavité de réception avec son extrémité de connexion en avant et ensuite lâché à l'intérieur de l'enveloppe souple 1 pour se positionner sous l'effet de la gravité sans contact de l'utilisateur avec l'outil d'entrainement 3 pour éviter toute contamination. Il est ainsi rendu plus aisé de réaliser cette manœuvre de positionnement de l'outil d'entraînement 3 si les première 13 et deuxième 12 ouvertures sont disposées à l'opposé l'une de l'autre.

L'enveloppe souple 1 forme en outre une portion annulaire 14 d'étanchéité disposée au voisinage de la portion d'extrémité 15, entre les première 13 et deuxième 12 ouvertures. De manière préférée, la portion annulaire 14 est contigüe à la portion d'extrémité 15.

En référence aux figures 1 et 2, la portion annulaire 14 est configurée pour être reçue au contact du boîtier 36 de l'outil d'entrainement 3 lorsque celui-ci est engagé dans la portion d'extrémité 15. En particulier, la portion annulaire 14 est configurée pour être mise au contact d'une surface annulaire 33 du boîtier 36 lorsque celui-ci est engagé dans la portion d'extrémité 15.

Cette surface de contact entre le boîtier 36 et l'enveloppe souple 1 permet d'éviter la propagation d'impuretés provenant de l'intérieur de l'enveloppe vers la deuxième ouverture 12. La portion annulaire 14 est de préférence ajustée sur la surface annulaire de manière à augmenter la surface de contact et ainsi réduire la possibilité de propagation des impuretés.

La portion annulaire 14 de l'enveloppe 1 et la surface annulaire 33 du boîtier 36 sont de préférence en complémentarité de forme de manière à améliorer encore davantage la surface de contact. De manière encore préférée, la portion annulaire 14 de l'enveloppe 1 présente une section transversale au moins partiellement inférieure à la section transversale de la surface annulaire 33 du boîtier 36. Ainsi, la portion annulaire 14 se déforme au contact de la surface annulaire 33 lorsque l'outil d'entrainement est inséré dans la portion d'extrémité 15. Cette déformation permet un ajustement serré entre la portion annulaire 14 et la surface annulaire 33 améliorant ainsi la capacité de l'enveloppe 1 à bloquer les impuretés.

De plus, la portion annulaire 14 et la surface annulaire 33 ont de préférence une section transversale conique, tel que visible sur les figures 1, 2 et 8. Ces sections transversales coniques sont orientées de telle sorte qu'elles descendent respectivement vers la deuxième ouverture 12 et l'embout de connexion 32. On entend par « descendre » le fait que la dimension de la section transversale décroit en direction de la deuxième ouverture 12 et de l'embout de connexion 32, respectivement.

Cette conformation conique permet un positionnement axial et longitudinal de l'outil d'entrainement 3 à l'intérieur de l'enveloppe 1.

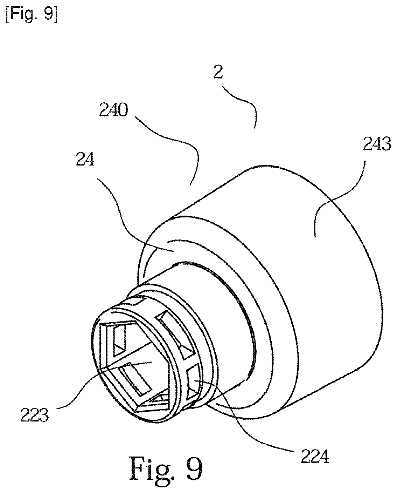

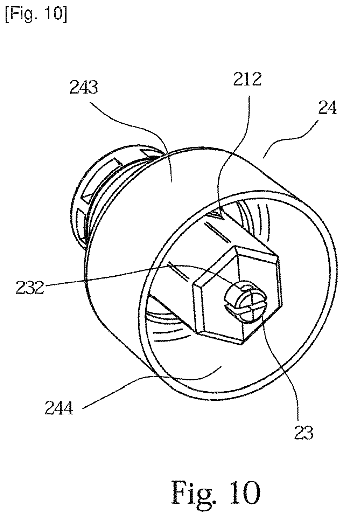

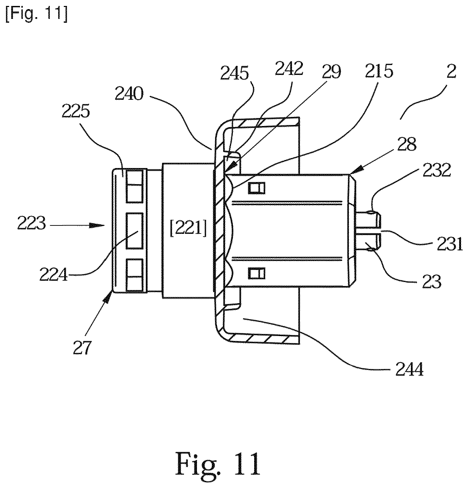

En référence aux figures 1 à 3 et 9 à 11, le coupleur 2 a pour fonction de permettre le couplage d'un instrument chirurgical percutané à l'outil d'entrainement 3 de manière à mettre en rotation cet instrument. La portion distale 27 forme ainsi une interface mécanique entre le coupleur 2 et l'extrémité d'un instrument chirurgical.

Le coupleur 2 est de préférence un élément de forme allongée présentant une symétrie de révolution autour de son axe longitudinal A.

Le coupleur 2 comprend une portion distale 27 configurée pour être connectée de manière amovible à un instrument chirurgical percutané.

La portion distale 27 comporte un corps cylindrique 221, de section externe circulaire, définissant une cavité interne formant une cavité d'accueil 223 destinée à recevoir l'extrémité de l'instrument chirurgical percutané auquel on veut accoupler l'outil d'entrainement 3.

Selon le mode de réalisation illustré, la portion distale 27 est destinée à être placée à l'extérieur de l'enveloppe 1 lorsque le coupleur 2 est connecté à l'outil d'entraînement 3 avec l'enveloppe 1 positionnée sur l'outil d'entraînement 3. La longueur la portion distale 27 est principalement définie de telle façon que, lorsque le coupleur 2 est mis en place, une extrémité libre 225 de la portion distale 27 est située à l'extérieur de l'enveloppe 1.

Dans la forme de réalisation prise comme exemple, la cavité d'accueil 223 est une cavité de section hexagonale sur les parois de laquelle sont ménagées, au niveau de l'extrémité 225, des ouvertures 224 permettant l'emboitage d'ergots de fixation placés à l'extrémité de l'instrument chirurgical percutané. L'embout d'extrémité de l'instrument chirurgical percutané peut alors, par exemple, prendre la forme d'un cylindre à base hexagonale, destiné à venir se loger dans une cavité cylindrique aux parois hexagonales, ménagée dans un moyen de mise en rotation donné.

La longueur de l'embout d'extrémité de l'instrument, la profondeur de la cavité, ainsi que les dimensions générales de la portion distale 27 et de la cavité d'accueil 223 sont principalement définies de telle façon que l'ensemble soit assemblé avec un minimum de jeu, de sorte que la mise en œuvre par le praticien dudit outil à embout rotatif entraine une rotation de l'instrument sur lui-même autour de son axe de symétrie et une pénétration de l'instrument selon une direction sensiblement rectiligne.

Le coupleur 2 comprend également une portion proximale 28 configurée pour être connectée de manière amovible à l'outil d'entrainement 3. En particulier, la portion proximale 28 est configurée pour être connectée de manière amovible à l'embout de connexion 32. La portion proximale 28 forme ainsi une interface mécanique entre le coupleur 2 et l'outil d'entraînement 3. La portion proximale 28 est destinée à venir se loger au moins partiellement à l'intérieur de l'enveloppe 1 lorsqu'elle est connectée à l'outil d'entraînement 3.

La portion proximale 28 est de préférence une connexion mâle configurée pour se connecter à l'embout de connexion 32 femelle de l'outil d'entraînement 3.

Dans l'exemple de réalisation considéré, une paroi externe de la portion proximale 28 forme un élément mâle de section hexagonale. Dans ce cas, l'embout de connexion 32 est également de section hexagonale de manière à pouvoir inséré la portion proximale 28 à l'intérieur de l'embout de connexion 32.

La portion proximale 28 peut également comprendre une zone tronconique 215 configurée pour coopérer avec une forme tronconique 322 de l'embout de connexion 32. L'emboitement de la zone tronconique 215 avec la forme tronconique 322 de l'embout de connexion 32 permet d'assurer le maintien sans jeu de la concentricité du coupleur 2 et de l'embout de connexion 32 lors de l'utilisation. Les zone 215 et forme 322 tronconiques ont également pour fonction de réaliser une butée en translation du coupleur 2 dans l'embout de connexion 32.

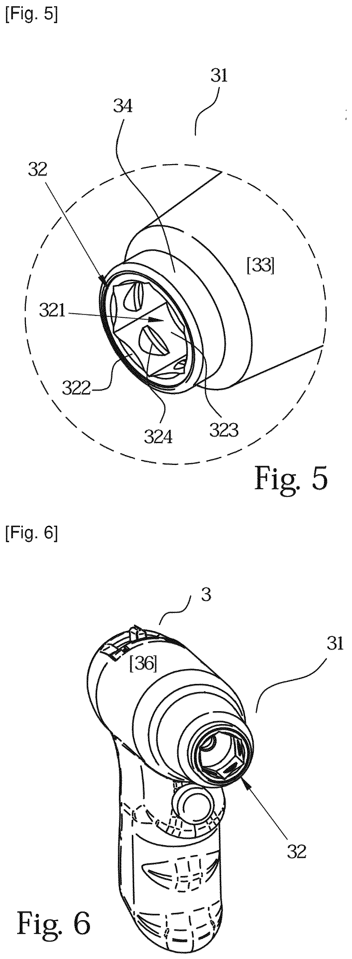

La portion proximale 28 peut également comprendre un organe de fixation du coupleur 2 à l'embout de connexion 32. En référence aux figures 5, 7, 10 et 11, l'organe de fixation peut comprendre un élément d'extrémité 23 cylindrique, fendu sur sa longueur et destiné à être inséré dans un perçage cylindrique borgne 325 formant le fond de l'embout de connexion 32 de l'outil d'entrainement 3.

L'élément d'extrémité 23 comprend de préférence une pluralité de bossages 232 formant des surépaisseurs telles que le diamètre de la section de l'élément d'extrémité 23 est localement supérieur au diamètre du perçage 325. La présence d'une fente 231 permet de rendre l'élément d'extrémité 23 déformable de telle façon que lors du couplage du coupleur 2 à l'embout de connexion 32 de l'outil d'entrainement 3, les bossages 232 frottent contre la paroi du perçage 325 ce qui permet avantageusement d'éviter que le coupleur 2 ne bouge librement dans l'embout de connexion 32.

L'organe de fixation peut également comprendre au niveau de la paroi externe de la portion proximale 28 une pluralité de bossages chanfreinés 212 conformés pour pouvoir se loger dans une gorge chanfreinée 324 réalisée dans une paroi interne de la cavité 321 de l'embout de connexion 32. Il est ainsi possible d'assurer le blocage du coupleur 2 par encliquetage de la portion proximale 28 dans l'embout de connexion 32.

Par ailleurs, la portion proximale 28 peut définir une cavité interne prolongeant la cavité 223 de la portion distale 27 de telle façon que l'embout de l'instrument chirurgical percutané puisse se loger totalement dans le coupleur 2. Cette cavité 223 est fermée du côté de l'extrémité de la portion proximale 28.

Le coupleur 2 comprend en outre une portion centrale 29 disposée entre les portions distale 27 et proximale 28. La portion centrale 29 forme une section du coupleur 2 disposé entre les portions distale 27 et proximale 28. Cette section peut être cylindrique circulaire et, par exemple, annulaire.

La portion centrale 29 comprend une collerette d'appui 240 s'étendant à partir de la section formée par la portion centrale 29. La collerette d'appui 240 s'étend transversalement à l'axe de couplage A. En d'autres termes, la collerette d'appui 240 s'étend sensiblement radialement à l'axe de couplage A. La collerette d'appui 240 forme une paroi de préférence circulaire s'étendant autour de la section de la portion centrale 29.

La collerette d'appui 240 est configurée pour déformer axialement l'extrémité distale de l'enveloppe souple 1 entourant l'outil d'entraînement 3 lorsque la portion proximale est connectée à cet outil d'entraînement 3. En d'autres termes, la collerette d'appui 240 est configurée pour venir s'appuyer contre l'extrémité distale de la portion d'extrémité 15 de l'enveloppe 1 pour la déformer et former un repli annulaire réalisant une étanchéité lorsque le coupleur est monté dans l'outil d'entraînement 3.

Pour cela, la collerette d'appui 240 est conformée de manière à ce que sa dimension externe maximale, i.e. son diamètre extérieur lorsque sa section est de forme circulaire, soit supérieure à la section de la portion proximale 28. En d'autres termes, la collerette d'appui 240 forme ainsi un disque ou une bride s'étendant radialement au-delà de la portion proximale 28. En particulier, cette dimension externe maximale, ou diamètre extérieur, est égale ou supérieure à la dimension externe maximale, ou diamètre extérieur, de l'extrémité de connexion 31 de l'outil d'entraînement 3.

De plus, l'enveloppe souple 1 est conformée de telle façon qu'une portion de l'enveloppe 1 formant la deuxième ouverture 12 s'étend au-delà de l'embout de connexion 32 lorsque la portion annulaire 14 de l'enveloppe 1 est au contact de la surface annulaire 33 de l'outil d'entraînement 3. Cette portion de l'enveloppe 1 est réalisée de telle sorte que sa longueur est supérieure à la distance prise suivant l'axe de couplage A séparant l'extrémité distale de l'embout de connexion 32 du coupleur 2. Ainsi, cette portion de l'enveloppe 1 rentre en contact avec la collerette d'appui 240 puis se déforme lorsque le coupleur 2 est accouplé à l'outil d'entraînement lorsque celui-ci est inséré dans l'enveloppe 1. Cette portion déformée de l'enveloppe réalise ainsi une barrière pour les impuretés potentielles provenant de l'intérieur de l'outil d'entraînement 3. En effet, les impuretés n'ont qu'un passage très exigu et tortueux pour sortir de l'enveloppe par la deuxième ouverture 12.

L'effet de cette déformation de l'extrémité de l'enveloppe 1est notamment visible en coupe sur les figures 1 et 2 et en vue de côté sur la figure 3.

De plus, lorsque l'outil d'entrainement 3 est logé dans l'enveloppe 1 et que le coupleur 2 est monté dans l'embout de connexion 32, les retours 16 situés au niveau des portions annulaire 14 et d'extrémité 15 se déforment et viennent en contact avec la surface interne de la collerette de maintien 243 (voir figure 2). Ceci permet lors de la rotation du coupleur 2 de retenir toutes les particules qui auraient pu s'échapper de l'étanchéité formée par la déformation de l'extrémité de la portion d'extrémité 15 contre la collerette d'appui 240.

La portion centrale 29 comprend également une collerette de maintien 243 s'étendant le long de l'axe de couplage A. La collerette de maintien 243 forme une paroi annulaire s'étendant le long de l'axe de couplage A. La collerette de maintien 243 s'étend de préférence à partir d'une extrémité de la collerette d'appui 240 de sorte que les collerettes d'appui 240 et de maintien 243 sont contiguës. La collerette de maintien 243 s'étend autour de l'axe de couplage A de manière à former une cloche de protection 24 configurée pour recouvrir l'extrémité de connexion 33 de l'outil d'entraînement 3. Ainsi, la dimension de la collerette de maintien 243 suivant l'axe de couplage A est définie pour que la collerette de maintien 243 recouvre cette extrémité de connexion 33. Pour cela, la collerette de maintien 243 recouvre au moins partiellement la portion proximale 28 de manière à pouvoir maintenir l'extrémité distale de l'enveloppe souple stérile entre la collerette de maintien 243 et le boîtier 36 de l'outil d'entraînement 3 lorsque la portion proximale 28 est connectée à l'outil d'entraînement 3.

La collerette de maintien 243 s'étend de préférence de manière à recouvrir au moins un tiers de la longueur de la portion annulaire 14 de l'enveloppe 1, le long de l'axe de couplage A, lorsque l'outil d'entrainement 3 est connecté au coupleur 2. De manière encore préférée, la collerette de maintien 243 s'étend de préférence de manière à recouvrir sensiblement la moitié de la longueur de cette portion annulaire 14 à l'état connecté de l'outil d'entraînement 3 et du coupleur 2.

La collerette de maintien 243 est réalisée de telle sorte que sa dimension transversale interne, ou diamètre intérieur lorsqu'elle est de section circulaire, est supérieure au diamètre extérieur de la partie d'extrémité de connexion 31 qu'elle recouvre. Ainsi, la dimension intérieure de la collerette de maintien 243 permet son insertion autour de l'extrémité de connexion 31 de l'outil d'entraînement 3.

La collerette de maintien 243 est de préférence cylindrique. La collerette de maintien 243 forme ainsi une section annulaire autour de la portion proximale 28. Un espace annulaire 244 est ainsi formé entre la collerette de maintien 243 et la portion proximale 28 pour recevoir une extrémité de l'enveloppe 1.

Les collerettes de maintien 243 et d'appui 240 ont de préférence la forme d'une cloche ou d'une section transversale en U. La base du U est réalisée par la collerette d'appui 240 et les bras du U sont réalisés par la collerette de maintien 243. Autrement dit, la portion centrale comprend un corps de liaison disposé entre les portions distale 27 et proximale 28, la collerette d'appui 240 s'étendant à partir du corps de liaison et formant avec la collerette de maintien 243 une forme générale de U.

Le coupleur 2 a de préférence une forme générale cylindrique ou tubulaire avec les collerettes maintien 243 et d'appui 240 formant une cloche ou un U autour de ce cylindre.

De plus, la portion d'extrémité 15 de l'enveloppe 1 a de préférence une dimension extérieure maximale inférieure à la dimension intérieure de la collerette de maintien 243 pour faciliter l'insertion de l'enveloppe 1 dans la collerette de maintien 243.

La collerette de maintien 243 comprend une extrémité distale disposée au niveau de la collerette d'appui 240 et une extrémité proximale libre. La collerette de maintien est de préférence conformée de manière à ce que l'extrémité proximale libre soit disposée à proximité du boîtier 36 lorsque le coupleur 2 est connecté à l'outil d'entraînement 3. Ainsi, les possibilités de propagation des impuretés hors de l'espace annulaire 244 formé par la collerette de maintien 243 sont réduites. Selon un exemple de réalisation, la collerette de maintien 243 et la surface annulaire 33 de l'outil d'entraînement 3 sont dimensionnées de manière à former un jeu inférieur ou égal à 3mm, de préférence inférieur ou égal à 2mm, entre l'extrémité proximale libre de la collerette de maintien 243 et la surface annulaire 33 de l'outil d'entraînement 3 lorsque l'outil d'entrainement 3 est connecté au coupleur 2.

Tel que visible en figures 1 et 2, un espace annulaire 244 est ainsi formé par la collerette de maintien 243 autour de la portion proximale 28. L'extrémité distale de l'outil d'entraînement 3 est ainsi confinée à l'intérieur de cet espace annulaire 244 lorsqu'il est connecté au coupleur 2. Ceci permet de canaliser les potentielles impuretés provenant de l'intérieur de l'enveloppe 1 ou de l'outil d'entraînement 3 dans cet espace annulaire 244. L'espace annuaire est fermé au niveau d'une première extrémité par la collerette d'appui 240 et ouvert à une deuxième extrémité opposée.

La portion centrale 29 comprend en outre une collerette de protection 242 s'étendant le long de l'axe de couplage A entre la collerette de maintien 243 et la portion proximale 28. En d'autres termes, la collerette de protection 242 s'étend à l'intérieur de l'espace annulaire 244 formé par la collerette de maintien 243. La collerette de protection 242 s'étend de préférence à partir de la collerette d'appui 240.

Cette collerette de protection 242 est configurée pour recouvrir au moins partiellement l'extrémité de connexion 31 de l'outil d'entraînement 3 lorsque le coupleur 2 est accouplé à l'outil d'entraînement 3. En d'autres termes, la collerette de protection 242 est configuré pour recouvrir une portion du boîtier 36 disposée au niveau de son extrémité de connexion.

Cette conformation de la collerette de protection 242 permet d'ajouter un obstacle pour les impuretés provenant de l'intérieur de l'outil d'entraînement 3. En effet, le passage pour ces impuretés est ainsi rendu davantage tortueux et permet de réduire les risques de sortie de ces impuretés par l'espace annuaire.

De plus, l'extension longitudinale de la collerette de protection 242 forme un espace de rétention 245 des impuretés entre la collerette de protection 242 et la portion proximale 28. En effet, les potentielles impuretés sortant de l'outil d'entraînement 3 sont projetées à l'intérieur de cet espace de rétention 245 sous l'effet de la force centrifuge générée par la rotation de l'embout de connexion 32.

La collerette de protection 242 est de préférence de section conique descendant vers la portion proximale 28. En d'autres termes, la dimension transversale de la collerette de protection 242 décroit depuis son extrémité proximale fixée à la collerette d'appui 240 vers son extrémité distale libre. Ainsi, la rétention des impuretés est encore améliorée.

Selon un mode de réalisation, le diamètre interne de la collerette de protection 242 est supérieur au diamètre externe de l'extrémité de connexion 34 du boîtier 36 de façon à ne pas générer de frottement lors de la mise en rotation du coupleur 2.

L'ensemble formé par les collerettes d'appui 240 et de maintien 243 peut être une pièce rapportée sur la portion centrale 29 du coupleur 2 ou bien monobloc avec cette portion centrale 29. La collerette de protection 242 est de préférence formée de manière monobloc avec la collerette d'appui 240. Ainsi, la collerette de protection 240 fait partie de cet ensemble formé par les collerettes d'appui 240 et de maintien 243.

Lorsque l'une ou plusieurs des collerettes d'appui 240, de protection 242 et de maintien 243 est rapportée, elle n'a pas vocation à être dissociée des portions proximale 28 et distale 27. Le coupleur 2 forme un accessoire unitaire dont l'agencement est définitif une fois fabriqué et assemblé. Aucune des collerettes d'appui 240, de protection 242 et de maintien 243 ne peut donc être dissociée puis assemblée provisoirement ou définitivement à un autre dispositif, par exemple l'enveloppe 1 ou le dispositif d'entrainement 3.

De manière préférée, la deuxième ouverture 12 et la portion d'extrémité 15 présentent un diamètre inférieur au diamètre interne minimal de la collerette de maintien 243 mais supérieur au diamètre extérieur maximal de la collerette de protection 242. Ceci afin d'éviter tout risque de blocage de la rotation du coupleur 2 par resserrement de la portion d'extrémité 15 autour de la collerette de protection 242 à cause des frottements de l'extrémité distale de la portion d'extrémité 15 sur la collerette d'appui 240.

Comme tend à le montrer la description qui précède, la structure du coupleur 2 et du dispositif chirurgical 10 selon l'invention permet ainsi avantageusement, en particulier dans la forme de réalisation préférée prise comme exemple, de garantir une étanchéité entre la cavité de réception de l'enveloppe 1 susceptible de renfermer un matériel non stérile (l'outil d'entrainement 3 en particulier) et le reste de l'espace dans lequel le dispositif est mise en œuvre, espace par principe stérile.

Compte tenu des caractéristiques techniques du coupleur 2 et du dispositif chirurgical 10 selon l'invention, il est ainsi avantageusement possible de réaliser de manière simple le couplage amovible d'un outil d'entrainement 3, outil non stérile, à un instrument de biopsie stérile, sans risquer de contaminer de manière involontaire le milieu stérile durant l'opération de couplage ou celle inverse de séparation.

Il est à considérer ici que compte tenu de la relation étroite existant entre les structures des extrémités respectives du coupleur 2 et de l'embout de connexion 32 de l'outil d'entrainement 3, destinées à coopérer pour assurer la liaison de ces deux éléments, l'invention a pour but de permettre le couplage amovible d'un outil d'entrainement 3, outil non stérile, à un instrument de biopsie nécessairement stérile en garantissant le confinement de l'outil d'entrainement 3, mais également d'assurer de manière optimale la mise en rotation de l'instrument de biopsie par l'outil d'entrainement 3. Par manière optimale on entend ici une manière permettant notamment de limiter les jeux pouvant exister au niveau des différentes interfaces formant la chaine mécanique reliant l'outil d'entrainement 3 et l'instrument de biopsie.

D'un point de vue opérationnel, l'opération de couplage stérile d'un instrument de biopsie et d'un outil d'entrainement dudit instrument au moyen du dispositif chirurgical 10 selon l'invention peut être décrite par les étapes d'implémentation suivantes :
- une première étape durant laquelle le praticien, équipé de gants stériles, saisit l'enveloppe 1 par les replis 17, l'ouvre et la présente à l'assistant qui insère l'extrémité de connexion 31 de l'outil d'entrainement 3 dans la portion d'extrémité 15 de l'enveloppe 1 et lâche l'outil d'entrainement 3, celui-ci étant alors maintenu et retenu par le praticien au moyen de l'enveloppe 1 ;
- une deuxième étape durant laquelle le praticien referme hermétiquement la première ouverture 13 de l'enveloppe 1 à l'aide de moyens de fermeture 18 disposés au niveau de ladite première ouverture 13 sans toucher à l'outil d'entrainement 3, de façon à ce qu'elle enferme l'outil d'entrainement 3;
- une troisième étape durant laquelle le praticien assemble l'embout de connexion 32 de l'outil d'entrainement 3 sur la portion proximale 28 du coupleur 2 jusqu'à réaliser l'emboitement des deux éléments l'un dans l'autre; la portion d'extrémité 15 est ainsi déformée par la collerette d'appui 240 à l'intérieur de l'espace annulaire 244 formé par la collerette de maintien 243 ;
- une quatrième étape durant laquelle le praticien met en place l'instrument chirurgical percutané en l'insérant dans la portion distale 27 du coupleur 2.

## Revendications

1. Coupleur (2) pour le couplage stérile d'un instrument chirurgical percutané à un outil d'entrainement (3) en rotation, s'étendant le long d'un axe de couplage (A), ledit coupleur (2) comprenant :
- une portion distale (27) configurée pour être connectée de manière amovible à un instrument chirurgical percutané,
- une portion proximale (28) configurée pour être connectée de manière amovible à un outil d'entrainement (3),
- une portion centrale (29) disposée entre les portions distale (27) et proximale (28), **caractérisé en ce que** la portion centrale (29) comprend :
∘une collerette d'appui (240) s'étendant transversalement à l'axe de couplage (A) et configurée pour déformer axialement une extrémité distale d'une enveloppe souple (1) entourant l'outil d'entraînement (3) lorsque la portion proximale (28) est connectée à cet outil d'entraînement (3),
∘ une collerette de maintien (243) s'étendant le long de l'axe de couplage (A) au moins partiellement en recouvrement de la portion proximale (28) de manière à pouvoir maintenir l'extrémité distale de l'enveloppe souple (1) stérile entre la collerette de maintien (243) et le boîtier (36) d'un outil d'entraînement (3) lorsque la portion proximale (28) est connectée à l'outil d'entraînement (3),
dans lequel la portion centrale comprend un corps de liaison disposé entre les portions distale (27) et proximale (28), la collerette d'appui (240) s'étendant à partir du corps de liaison et formant avec la collerette de maintien (243) une forme générale de U, la collerette de maintien (243) s'étendant à partir d'une extrémité de la collerette d'appui (240) pour former un espace annulaire (244) autour de la portion proximale (28),
dans lequel le coupleur forme un accessoire unitaire dans lequel les portions distale (27), proximale (28) et centrale (29) sont solidaires les unes des autres.

2. Coupleur (2) selon la revendication 1, dans lequel les portions distale (27), proximale (28) et centrale (29) sont fixes les unes par rapport aux autres.

3. Coupleur (2) selon la revendication 1 ou 2, dans lequel la collerette de maintien (243) s'étend autour de l'axe de couplage (A) de manière à former une cloche de protection (24) configurée pour recouvrir une extrémité de connexion (31) d'un outil d'entraînement (3).

4. Coupleur (2) selon l'une quelconque des revendications 1 à 3, dans lequel la portion centrale (29) comprend en outre une collerette de protection (242) s'étendant le long de l'axe de couplage (A) entre la collerette de maintien (243) et la portion proximale (29).

5. Coupleur (2) selon l'une quelconque des revendications 1 à 4, dans lequel la portion proximale (28) est une connexion mâle configurée pour se connecter à un embout de connexion (32) femelle d'un outil d'entraînement (3).

6. Dispositif chirurgical (10) stérile, comprenant :
- un coupleur (2) selon l'une quelconque des revendications précédentes,
- une enveloppe (1) souple formant :
∘ une cavité de réception d'un outil d'entraînement (3),
∘une première ouverture (13) configurée pour permettre l'insertion de l'outil d'entraînement (3) à l'intérieur de la cavité de réception,
∘ une portion d'extrémité (15) configurée pour recevoir une extrémité de connexion (31) de l'outil d'entraînement (3), la portion d'extrémité (15) formant une deuxième ouverture (12) débouchant à l'intérieur de la cavité de réception et configurée pour permettre l'insertion partielle du coupleur (2) à l'intérieur de la cavité de réception au travers de la deuxième ouverture (12),
∘ une portion annulaire (14),
la collerette de maintien (243) du coupleur étant configurée pour recouvrir au moins partiellement la portion annulaire (14) de l'enveloppe (1) lorsque l'extrémité de connexion de l'outil d'entraînement est insérée dans la portion d'extrémité (15) de l'enveloppe (1) avec la portion proximale du coupleur connectée à l'embout de connexion (32).

7. Dispositif chirurgical (10) selon la revendication 6, comprenant en outre :
- un outil d'entraînement (3) comprenant :
∘ un boîtier (36) et un organe d'entrainement en rotation disposé à l'intérieur du boîtier (36), le boîtier (36) définissant une surface annulaire (33) de réception d'une enveloppe (1) souple au niveau d'une extrémité de connexion (31) de l'outil d'entraînement (3),
∘ un embout de connexion (32) disposé au niveau de l'extrémité de connexion (31) et configuré pour être connecté de manière amovible à la portion proximale (28) du coupleur (2), l'embout de connexion (32) étant solidaire de l'organe d'entrainement de manière à transmettre au coupleur (2) un mouvement de rotation de l'organe d'entrainement lorsque la portion proximale (28) est connectée à l'embout de connexion (32).

8. Dispositif chirurgical (10) selon la revendication 7, dans lequel la portion annulaire (14) est configurée pour être reçue au contact de la surface annulaire (33) du boîtier de l'outil d'entraînement (3) lorsque l'extrémité de connexion (31) de l'outil d'entraînement (3) est insérée dans la portion d'extrémité (15) de l'enveloppe (1).

9. Dispositif chirurgical (10) selon la revendication 8, dans lequel la portion annulaire (14) de l'enveloppe (1) et la surface annulaire (33) du boîtier (36) de l'outil d'entraînement (3) sont en complémentarité de forme.

10. Dispositif chirurgical (10) selon la revendication 8 ou 9, dans lequel la portion annulaire (14) de l'enveloppe (1) présente une section transversale au moins partiellement inférieure à la section transversale de la surface annulaire (33) du boîtier (36) de l'outil d'entraînement (3) de manière à déformer l'enveloppe (1) lorsque l'outil d'entrainement (3) est inséré dans la portion d'extrémité (15) avec la portion annulaire (14) et la surface annulaire (33) en contact l'une avec l'autre.

11. Dispositif chirurgical (10) selon la revendication 9ou 10, dans lequel la portion annulaire (14) et la surface annulaire (33) ont une section transversale conique descendant respectivement vers la deuxième ouverture (12) et l'embout de connexion (32).

12. Dispositif chirurgical (10) selon l'une quelconque des revendications 6 à 11, dans lequel la portion annulaire (14) de l'enveloppe (1) est contiguë à la portion d'extrémité (15).

13. Dispositif chirurgical (10) selon l'une quelconque des revendications 6 à 12, dans lequel l'enveloppe (1) est formée par au moins deux feuillets fixées entre eux au niveau de leurs bords d'extrémité pour former la cavité de réception, un retour (16) sensiblement transversal aux feuillets étant formé le long des bords d'extrémité lorsque l'outil d'entrainement (3) est logé dans ladite enveloppe (1).

14. Dispositif chirurgical (10) selon la revendication 13, dans lequel lesdits au moins deux feuillets sont fixés ensemble par soudage.

15. Dispositif chirurgical (10) selon l'une quelconque des revendications 6 à 14, dans lequel la collerette de maintien (243) s'étend de manière à recouvrir au moins un tiers de la longueur de la portion annulaire (14) le long de l'axe de couplage (A) lorsque l'outil d'entrainement (3) est connecté au coupleur (2).

16. Dispositif chirurgical (10) selon l'une quelconque des revendications 6 à 15 en combinaison avec la revendication 9, dans lequel la collerette de maintien (243) comprend une extrémité distale disposée au niveau de la portion centrale (29) du coupleur (2) et une extrémité proximale libre, la collerette de maintien (243) et la surface annulaire (33) de l'outil d'entraînement (3) étant dimensionnées de manière à former un jeu inférieur ou égal à 3mm, de préférence inférieur ou égal à 2mm, entre l'extrémité proximale de la collerette de maintien (243) et la surface annulaire (33) de l'outil d'entraînement (3) lorsque l'outil d'entrainement (3) est connecté au coupleur (2).

17. Dispositif chirurgical (10) selon l'une quelconque des revendications 6 à 16, dans lequel l'enveloppe (1) est dépourvue de pièce rigide de connexion à l'outil d'entraînement (3) ou au coupleur (2), à l'état déconnecté de l'outil d'entraînement (3), du coupleur (2) et de l'enveloppe (1).

## Patentansprüche

1. Koppler (2) zum sterilen Koppeln eines perkutanen chirurgischen Instruments mit einem rotierenden Antriebswerkzeug (3), das sich entlang einer Kopplungsachse (A) erstreckt, wobei der Koppler (2) Folgendes umfasst:
- einen distalen Abschnitt (27), der dazu ausgebildet ist, lösbar mit einem perkutanen chirurgischen Instrument verbunden zu werden,
- einen proximalen Abschnitt (28), der dazu ausgebildet ist, lösbar mit einem Antriebswerkzeug (3) verbunden zu werden,
- einen zwischen dem distalen Abschnitt (27) und dem proximalen Abschnitt (28) angeordneten mittleren Abschnitt (29), **dadurch gekennzeichnet, dass** der mittlere Abschnitt (29) Folgendes umfasst:
∘ einen Anlageflansch (240), der sich quer zur Kopplungsachse (A) erstreckt und dazu ausgebildet ist, ein distales Ende einer flexiblen Hülle (1), welche das Antriebswerkzeug (3) umgibt, axial zu verformen, wenn der proximale Abschnitt (28) mit diesem Antriebswerkzeug (3) verbunden ist,
∘ einen Haltekragen (243), der sich entlang der Kopplungsachse (A) erstreckt und den proximalen Abschnitt (28) zumindest teilweise überdeckt, sodass das distale Ende der sterilen flexiblen Hülle (1) zwischen dem Haltekragen (243) und dem Gehäuse (36) eines Antriebswerkzeugs (3) gehalten werden kann, wenn der proximale Abschnitt (28) mit dem Antriebswerkzeug (3) verbunden ist,
wobei der mittlere Abschnitt einen zwischen dem distalen Abschnitt (27) und dem proximalen Abschnitt (28) angeordneten Verbindungskörper umfasst, wobei sich der Anlageflansch (240) von dem Verbindungskörper aus erstreckt und zusammen mit dem Haltekragen (243) eine im Wesentlichen U-förmige Gestalt bildet, wobei sich der Haltekragen (243) von einem Ende des Anlageflansches (240) aus erstreckt, um einen ringförmigen Raum (244) um den proximalen Abschnitt (28) zu bilden,
wobei der Koppler ein einteiliges Zubehörteil bildet, bei dem der distale Abschnitt (27), der proximale Abschnitt (28) und der mittlere Abschnitt (29) fest miteinander verbunden sind.

2. Koppler (2) nach Anspruch 1, wobei der distale Abschnitt (27), der proximale Abschnitt (28) und der mittlere Abschnitt (29) relativ zueinander feststehend sind.

3. Koppler (2) nach Anspruch 1 oder 2, wobei sich der Haltekragen (243) um die Kopplungsachse (A) herum erstreckt, sodass eine Schutzglocke (24) gebildet wird, die dazu ausgebildet ist, ein Verbindungsende (31) eines Antriebswerkzeugs (3) zu überdecken.

4. Koppler (2) nach einem der Ansprüche 1 bis 3, wobei der mittlere Abschnitt (29) ferner einen Schutzkragen (242) umfasst, der sich entlang der Kopplungsachse (A) zwischen dem Haltekragen (243) und dem proximalen Abschnitt (29) erstreckt.

5. Koppler (2) nach einem der Ansprüche 1 bis 4, wobei der proximale Abschnitt (28) einen männlichen Anschluss bildet, der dazu ausgebildet ist, mit einem weiblichen Verbindungsaufsatz (32) eines Antriebswerkzeugs (3) verbunden zu werden.

6. Sterile chirurgische Vorrichtung (10), umfassend:
- einen Koppler (2) nach einem der vorhergehenden Ansprüche,
- eine flexible Hülle (1), die Folgendes bildet:
∘ einen Aufnahmeraum zur Aufnahme eines Antriebswerkzeugs (3),
∘ eine erste Öffnung (13), die dazu ausgebildet ist, das Einführen des Antriebswerkzeugs (3) in den Aufnahmeraum zu ermöglichen,
∘ einen Endabschnitt (15), der dazu ausgebildet ist, ein Verbindungsende (31) des Antriebswerkzeugs (3) aufzunehmen, wobei der Endabschnitt (15) eine zweite Öffnung (12) bildet, die in den Aufnahmeraum mündet und dazu ausgebildet ist, ein teilweises Einführen des Kopplers (2) in den Aufnahmeraum durch die zweite Öffnung (12) zu ermöglichen,
∘ einen Ringabschnitt (14),
wobei der Haltekragen (243) des Kopplers dazu ausgebildet ist, den Ringabschnitt (14) der Hülle (1) zumindest teilweise zu überdecken, wenn das Verbindungsende des Antriebswerkzeugs in den Endabschnitt (15) der Hülle (1) eingeführt ist und der proximale Abschnitt des Kopplers mit dem Verbindungsaufsatz (32) verbunden ist.

7. Chirurgische Vorrichtung (10) nach Anspruch 6, ferner umfassend:
- ein Antriebswerkzeug (3), das Folgendes umfasst:
∘ ein Gehäuse (36) und ein im Gehäuse (36) angeordnetes Rotationsantriebselement, wobei das Gehäuse (36) an einem Verbindungsende (31) des Antriebswerkzeugs (3) eine Ringfläche (33) zur Aufnahme einer flexiblen Hülle (1) definiert,
∘ einen am Verbindungsende (31) angeordneten Verbindungsaufsatz (32), der dazu ausgebildet ist, lösbar mit dem proximalen Abschnitt (28) des Kopplers (2) verbunden zu werden, wobei der Verbindungsaufsatz (32) mit dem Antriebselement fest verbunden ist, um auf den Koppler (2) eine Rotationsbewegung des Antriebselements zu übertragen, wenn der proximale Abschnitt (28) mit dem Verbindungsaufsatz (32) verbunden ist.

8. Chirurgische Vorrichtung (10) nach Anspruch 7, wobei der Ringabschnitt (14) dazu ausgebildet ist, in Anlage an der Ringfläche (33) des Gehäuses des Antriebswerkzeugs (3) aufgenommen zu werden, wenn das Verbindungsende (31) des Antriebswerkzeugs (3) in den Endabschnitt (15) der Hülle (1) eingeführt ist.

9. Chirurgische Vorrichtung (10) nach Anspruch 8, wobei der Ringabschnitt (14) der Hülle (1) und die Ringfläche (33) des Gehäuses (36) des Antriebswerkzeugs (3) formkomplementär zueinander ausgebildet sind.

10. Chirurgische Vorrichtung (10) nach Anspruch 8 oder 9, wobei der Ringabschnitt (14) der Hülle (1) einen Querschnitt aufweist, der zumindest teilweise kleiner ist als der Querschnitt der Ringfläche (33) des Gehäuses (36) des Antriebswerkzeugs (3), sodass die Hülle (1) verformt wird, wenn das Antriebswerkzeug (3) in den Endabschnitt (15) eingeführt wird und der Ringabschnitt (14) sowie die Ringfläche (33) miteinander in Kontakt stehen.

11. Chirurgische Vorrichtung (10) nach Anspruch 9 oder 10, wobei der Ringabschnitt (14) und die Ringfläche (33) jeweils einen konischen Querschnitt aufweisen, der sich in Richtung der zweiten Öffnung (12) bzw. des Verbindungsaufsatzes (32) verjüngt.

12. Chirurgische Vorrichtung (10) nach einem der Ansprüche 6 bis 11, wobei der Ringabschnitt (14) der Hülle (1) an den Endabschnitt (15) angrenzt.

13. Chirurgische Vorrichtung (10) nach einem der Ansprüche 6 bis 12, wobei die Hülle (1) durch mindestens zwei miteinander verbundene Folienlagen gebildet ist, die an ihren Endrändern miteinander verbunden sind, um den Aufnahmeraum zu bilden, wobei entlang der Endränder ein im Wesentlichen quer zu den Folienlagen verlaufender Umschlag (16) gebildet wird, wenn das Antriebswerkzeug (3) in der Hülle (1) aufgenommen ist.

14. Chirurgische Vorrichtung (10) nach Anspruch 13, wobei die mindestens zwei Folienlagen durch Schweißen miteinander verbunden sind.

15. Chirurgische Vorrichtung (10) nach einem der Ansprüche 6 bis 14, wobei sich der Haltekragen (243) derart erstreckt, dass er mindestens ein Drittel der Länge des Ringabschnitts (14) entlang der Kopplungsachse (A) überdeckt, wenn das Antriebswerkzeug (3) mit dem Koppler (2) verbunden ist.

16. Chirurgische Vorrichtung (10) nach einem der Ansprüche 6 bis 15 in Kombination mit Anspruch 9, wobei der Haltekragen (243) ein am mittleren Abschnitt (29) des Kopplers (2) angeordnetes distales Ende und ein freies proximales Ende umfasst, wobei der Haltekragen (243) und die Ringfläche (33) des Antriebswerkzeugs (3) derart dimensioniert sind, dass zwischen dem proximalen Ende des Haltekragens (243) und der Ringfläche (33) des Antriebswerkzeugs (3) ein Spiel von höchstens 3 mm, vorzugsweise höchstens 2 mm, gebildet wird, wenn das Antriebswerkzeug (3) mit dem Koppler (2) verbunden ist.

17. Chirurgische Vorrichtung (10) nach einem der Ansprüche 6 bis 16, wobei die Hülle (1) im getrennten Zustand des Antriebswerkzeugs (3), des Kopplers (2) und der Hülle (1) keinen starren Verbindungsteil zur Verbindung mit dem Antriebswerkzeug (3) oder dem Koppler (2) ist.

## Claims

1. A coupler (2) for sterile coupling of a percutaneous surgical instrument to a rotational drive tool (3) extending along a coupling axis (A), said coupler (2) comprising:
- a distal portion (27) configured to be removably connected to a percutaneous surgical instrument,
- a proximal portion (28) configured to be removably connected to a drive tool (3),
- a central portion (29) disposed between the distal (27) and proximal (28) portions, **characterized in that** the central portion (29) comprises:
∘ a bearing flange (240) extending transversely to the coupling axis (A) and configured to axially deform a distal end of a flexible wrapper (1) surrounding the drive tool (3) when the proximal portion (28) is connected to said drive tool (3),
∘ a holding flange (243) extending along the coupling axis (A) at least partially overlapping the proximal portion (28) so as to be able to hold the distal end of the sterile flexible wrapper (1) between the holding flange (243) and the housing (36) of a drive tool (3) when the proximal portion (28) is connected to the drive tool (3),
wherein the central portion comprises a connecting body disposed between the distal (27) and proximal (28) portions, the bearing flange (240) extending from the connecting body and forming a general U-shape with the holding flange (243), the holding flange (243) extending from one end of the bearing flange (240) to form an annular space (244) around the proximal portion (28), wherein the coupler forms a unitary accessory wherein the distal (27), proximal (28) and central (29) portions are secured to one another.

2. The coupler (2) according to claim 1, wherein the distal (27), proximal (28) and central (29) portions are fixed relative to each other.

3. The coupler (2) according to claim 1 or 2, wherein the holding flange (243) extends around the coupling axis (A) so as to form a protective bell (24) configured to cover a connecting end (31) of a drive tool (3).

4. The coupler (2) according to any one of claims 1 to 3, wherein the central portion (29) further comprises a protective flange (242) extending along the coupling axis (A) between the holding flange (243) and the proximal portion (29).

5. The coupler (2) according to any one of claims 1 to 4, wherein the proximal portion (28) is a male connector configured to connect to a female connector endpiece (32) of a drive tool (3).

6. A sterile surgical device (10), comprising:
- a coupler (2) according to any one of the previous claims,
- a flexible wrapper (1) forming:
∘ a cavity for receiving a drive tool (3),
∘ a first opening (13) configured to allow the insertion of the drive tool (3) inside the receiving cavity,
∘ an end portion (15) configured to receive a connecting end (31) of the drive tool (3), the end portion (15) forming a second opening (12) that opens inside the receiving cavity and is configured to allow partial insertion of the coupler (2) inside the receiving cavity through the second opening (12),
∘ an annular portion (14),
the holding flange (243) of the coupler being configured to at least partially cover the annular portion (14) of the wrapper (1) when the connection end of the drive tool is inserted into the end portion (15) of the wrapper (1) with the proximal portion of the coupler connected to the connecting endpiece (32).

7. The surgical device (10) according to claim 6, further comprising:
- a drive tool (3) comprising:
∘ a housing (36) and a rotational drive member disposed inside the housing (36), the housing (36) defining an annular surface (33) for receiving a flexible wrapper (1) at a connecting end (31) of the drive tool (3),
∘ a connecting endpiece (32) disposed at the connecting end (31) and configured to be removably connected to the proximal portion (28) of the coupler (2), the connecting endpiece (32) being secured to the drive tool so as to transmit to the coupler (2) a rotational movement of the drive member when the proximal portion (28) is connected to the connecting endpiece (32).

8. The surgical device (10) according to claim 7, wherein the annular portion (14) is configured to be received in contact with the annular surface (33) of the housing of the drive tool (3) when the connection end (31) of the drive tool (3) is inserted into the end portion (15) of the wrapper (1).

9. The surgical device (10) according to claim 8, wherein the annular portion (14) of the wrapper (1) and the annular surface (33) of the housing (36) of the drive tool (3) are form-fit.

10. The surgical device (10) according to claim 8 or 9, wherein the annular portion (14) of the wrapper (1) has a cross-section at least partially smaller than the cross-section of the annular surface (33) of the housing (36) of the drive tool (3), so as to deform the wrapper (1) when the drive tool (3) is inserted into the end portion (15) with the annular portion (14) and the annular surface (33) in contact with each other.

11. The surgical device (10) according to claim 9 or 10, wherein the annular portion (14) and the annular surface (33) have a conical cross-section descending respectively towards the second opening (12) and the connecting endpiece (32).

12. The surgical device (10) according to any one of claims 6 to 11, wherein the annular portion (14) of the wrapper (1) is contiguous with the end portion (15).

13. The surgical device (10) according to any one of claims 6 to 12, wherein the wrapper (1) is formed by at least two sheets fixed together at their end edges to form the receiving cavity, a return (16) substantially transverse to the sheets being formed along the end edges when the drive tool (3) is housed in said wrapper (1).

14. The surgical device (10) according to claim 13, wherein said at least two sheets are fixed together by welding.

15. The surgical device (10) according to any one of claims 6 to 14, wherein the holding flange (243) extends so as to cover at least one third of the length of the annular portion (14) along the coupling axis (A) when the drive tool (3) is connected to the coupler (2).

16. The surgical device (10) according to any one of claims 6 to 15 in combination with claim 9, in which the holding flange (243) comprises a distal end disposed at the central portion (29) of the coupler (2) and a free proximal end, the holding flange (243) and the annular surface (33) of the drive tool (3) being dimensioned such that they form a clearance of 3 mm or less, preferably 2 mm or less, between the proximal end of the holding flange (243) and the annular surface (33) of the drive tool (3) when the drive tool (3) is connected to the coupler (2) .

17. The surgical device (10) according to any one of claims 6 to 16, wherein the wrapper (1) has no rigid part for connection to the drive tool (3) or to the coupler (2), in the disconnected state of the drive tool (3), the coupler (2) and the wrapper (1)\.
